(19) Europäisches Patentamt
European Patent Office
Office européen des brevets

(11) Veröffentlichungsnummer: **0 166 856**
**A1**

(12) # EUROPÄISCHE PATENTANMELDUNG

(21) Anmeldenummer: **85102074.3**

(22) Anmeldetag: **26.02.85**

(51) Int. Cl.⁴: **A 61 K 6/02**
A 61 K 6/00

(30) Priorität: **04.07.84 DE 3424538**

(43) Veröffentlichungstag der Anmeldung:
**08.01.86 Patentblatt 86/2**

(84) Benannte Vertragsstaaten:
**AT CH DE FR GB LI SE**

(71) Anmelder: **Kulzer & Co. GmbH**
**Philipp-Reis-Strasse 8**
**D-6393 Wehrheim (TS.)1(DE)**

(72) Erfinder: **Angrick, Michael, Dr.**
**Richard-Wagner-Strasse 45**
**D-1000 Berlin 10(DE)**

(72) Erfinder: **Späth, Martin Willi**
**Philipp-Reis-Strasse 8**
**D-6393 Wehrheim (Taunus) 1(DE)**

(74) Vertreter: **Heinen, Gerhard, Dr.**
**W.C. Heraeus GmbH Zentralbereich Patente und Lizenzen Heraeusstrasse 12-14**
**D-6450 Hanau(DE)**

(54) **Isoliermittel.**

(57) Isoliermittel aus wässerigen Alginat-Lösungen, die Konservierungsmittel enthalten und einen pH-Wert zwischen 5,5 und 8,5 besitzen, sind lagerstabil und weisen gleichbleibend gute Verarbeitungseigenschaften auch bei Aufbewahrung über einen längeren Zeitraum auf. Sie eignen sich zum Isolieren der Oberfläche von Gipsformen gegen Kunststoff.

EP 0 166 856 A1

0166856

Hanau, 26. Juni 1984
ZPL-Pr/ha

Kulzer & Co. GmbH

Patentanmeldung

"Isoliermittel"

Die Erfindung betrifft ein Isoliermittel aus einer ein Konservierungsmittel enthaltenden wässerigen Alginat-Lösung.

Bei der Herstellung von Kunststoff-Zahnprothesen und -Zahnersatzteilen werden für die Isolierung der Gipsoberfläche gegen den Kunststoff häufig Isoliermittel aus dünnflüssigen wässerigen Lösungen wasserlöslicher Alginate verwendet.

Ein Isoliermittel dieser Art wird zum Beispiel in der deutschen Patentschrift 811 713 beschrieben. Es besteht aus einer wässerigen Lösung von Natrium- oder Kaliumalginat, die gegebenenfalls noch Glycerin oder eine andere hygroskopische Substanz enthalten kann.

Die Haltbarkeit der gebrauchsfertigen Isoliermittel-Lösungen läßt sich durch den Zusatz von Konservierungsmitteln verbessern (deutsche Patentschrift 837 147; rumänisches Patent 71 677, referiert in CA 96, 110192; Stomatologiya, Sofia, 49, 344-53, 1967, referiert in CA 68, 6163).

Damit sich die Kunststoff-Zahnprothesen ohne Schwierigkeiten von der Gipsform ablösen lassen, wird in der deutschen Patentschrift 966 997 vorgeschlagen, wasserlösliche Amine enthaltende Alginat-Lösungen zu verwenden. Die Filmbildung dieser Amin-Alginat-Lösungen auf der Gipsoberfläche kann durch Zusatz von zum Beispiel Natriumcarbonat - als sogenannter Verzögerer auch in Alginat-Abformmaterialien enthalten - verzögert werden.

Es ist die Aufgabe der Erfindung, eine für die Isolierung der Gipsoberfläche gegen den Zahnprothesen-Kunststoff geeignete und gegen den Befall durch Mikroorganismen beständige wässerige Lösung eines Alginats zu finden, die über lange Zeit lagerstabil ist.

Das die Lösung der Aufgabe darstellende Isoliermittel mit Konservierungsmittel-Zusatz ist erfindungsgemäß dadurch gekennzeichnet, daß es ein Puffergemisch enthält und einen pH-Wert zwischen 5,5 und 8,5 besitzt.

Als Puffergemische haben sich Citronensäure/Natriumhydroxid (pH 6,5), Glycin/Natriumhydroxid (pH 8) und Dinatriumhydrogenphosphat-dihydrat/Kaliumdihydrogenphosphat (pH 8,5) besonders bewährt.

Isoliermittel gemäß der Erfindung besitzen eine ausgezeichnete Haltbarkeit und Lagerstabilität und behalten ihre guten Verarbeitungseigenschaften auch bei Aufbewahrung über einen längeren Zeitraum bei. Viskosität und pH-Wert der Isoliermittel bleiben über lange Zeit hinweg praktisch konstant.

Um die Gipsoberfläche zu aktivieren und jegliche Weißverfärbung des Kunststoffs auszuschließen, hat es sich bewährt, dem Isoliermittel ein wasserlösliches Kupfer(II)-Salz, zum Beispiel Kupfer(II)-chlorid, vorzugsweise in einer Menge von 0,1 - 0,2 Gewichts-%, und gegebenenfalls ein wasserlösliches Salz der Barbitursäure oder eines Barbitursäure-Derivats, zum Beispiel das Natriumsalz der 5-Phenylbarbitursäure, vorzugsweise in einer Menge von etwa 0,5 Gewichts-%, zuzusetzen.

Das Isoliermittel enthält ein oder mehrere der für diesen Zweck bekannten Konservierungsmittel, zum Beispiel Benzoesäure beziehungsweise Benzoate, p-Hydroxybenzoesäureester (PHB-Ester), Phenole, Sorbinsäure und ihre Salze, Formalin, Hexetidin, Natriumsalicylat und Neomycinsulfat, in zum Schutz gegen den Befall durch Mikroorganismen ausreichender Menge.

Die Isoliermittel sind 2 - 5 prozentige, vorzugsweise 2 - 3 prozentige wässerige Lösungen von Natrium-, Kalium- oder Ammoniumalginaten.

Zur näheren Erläuterung der Erfindung werden in den folgenden Beispielen Isoliermittel gemäß der Erfindung beschrieben.

Beispiel 1

Isoliermittel mit einem pH-Wert von 8,5

| | | |
|---|---|---|
| 96,2 | Gewichts-% | Wasser |
| 2,6 | " | Alginat (Manucol FH der Firma Alginat Industries GmbH, Hamburg) |
| 1 | " | Dinatriumhydrogenphosphat-dihydrat |
| 0,1 | " | Kaliumdihydrogenphosphat |
| 0,1 | " | Neomycinsulfat |

Beispiel 2

Isoliermittel mit einem pH-Wert von 6

| | | |
|---|---|---|
| 95,26 | Gewichts-% | Wasser |
| 0,6 | " | Natriumhydroxid |
| 1 | " | Citronensäure |
| 0,1 | " | Kupfer(II)-chlorid |
| 2,6 | " | Alginat (Manucol FH der Firma Alginat Industries GmbH, Hamburg) |
| 0,2 | " | Natriumpyrophosphat |

| | | |
|---|---|---|
| 0,12 | Gewichts-% | Sorbinsäure |
| 0,12 | " | Formalin, 37 %ig |

Beispiel 3

Isoliermittel mit einem pH-Wert von 6,5

| | | |
|---|---|---|
| 95,38 | Gewichts-% | Wasser |
| 0,6 | " | Natriumhydroxid |
| 1 | " | Citronensäure |
| 0,1 | " | Kupfer(II)-chlorid |
| 2,6 | " | Alginat (Manucol FH der Firma Alginat Industries GmbH, Hamburg) |
| 0,2 | " | Natriumpyrophosphat |
| 0,12 | " | Formalin, 37 %ig |

Beispiel 4

Isoliermittel mit einem pH-Wert von 8,5

| | | |
|---|---|---|
| 95,9 | Gewichts-% | Wasser |
| 2,6 | " | Alginat (Manucol FH der Firma Alginat Industries GmbH, Hamburg) |
| 1 | " | Dinatriumhydrogenphosphat-dihydrat |
| 0,1 | " | Kaliumdihydrogenphosphat |
| 0,2 | " | Natriumpyrophosphat |
| 0,1 | " | Kupfer(II)-chlorid |
| 0,1 | " | Neomycinsulfat |

Beispiel 5

Isoliermittel mit einem pH-Wert von 6,5

| | | |
|---|---|---|
| 95,4 | Gewichts-% | Wasser |
| 2,6 | " | Alginat (Manucol FH der Firma Alginat Industries GmbH, Hamburg) |

| | | |
|---|---|---|
| 1 | Gewichts-% | Citronensäure |
| 0,6 | " | Natriumhydroxid |
| 0,2 | " | Natriumpyrophosphat |
| 0,1 | " | Kupfer(II)-chlorid |
| 0,1 | " | Neomycinsulfat |

Beispiel 6

Isoliermittel mit einem pH-Wert von 8

| | | |
|---|---|---|
| 95,78 | Gewichts-% | Wasser |
| 0,7 | " | Glycin |
| 0,5 | " | Natriumchlorid |
| 0,02 | " | Natriumhydroxid |
| 2,6 | " | Alginat (Manuol FH der Firma Alginat Industries GmbH, Hamburg) |
| 0,2 | " | Natriumpyrophosphat |
| 0,1 | " | Kupfer(II)-chlorid |
| 0,1 | " | Neomycinsulfat |

Beispiel 7

Isoliermittel mit einem pH-Wert von 8

| | | |
|---|---|---|
| 95,24 | Gewichts-% | Wasser |
| 0,05 | " | Natriumhydroxid |
| 0,63 | " | Glycin |
| 0,49 | " | Natriumchlorid |
| 0,51 | " | Natriumsalz der 5-Phenylbarbitursäure |
| 0,13 | " | Kupfer(II)-chlorid |
| 2,64 | " | Alginat (Manucol FH der Firma Alginat Industries GmbH, Hamburg) |
| 0,2 | " | Natriumpyrophosphat |
| 0,11 | " | Formalin, 37 %ig |

Beispiel 8

Isoliermittel mit einem pH-Wert von 6,5

| | | |
|---|---|---|
| 94,84 | Gewichts-% | Wasser |
| 0,58 | " | Natriumhydroxid |
| 1,01 | " | Citronensäure |
| 0,51 | " | Natriumsalz der 5-Phenylbarbitursäure |
| 0,13 | " | Kupfer(II)-chlorid |
| 0,20 | " | Natriumpyrophosphat |
| 2,62 | " | Alginat (Manucol FH der Firma Alginat Industries GmbH, Hamburg) |
| 0,11 | " | Formalin, 37 %ig |

Beispiel 9

Isoliermittel mit einem pH-Wert von 6,5

| | | |
|---|---|---|
| 95,19 | Gewichts-% | Wasser |
| 0,67 | " | Glycin |
| 0,02 | " | Natriumhydroxid |
| 0,55 | " | Natriumchlorid |
| 0,5 | " | Natriumsalz der 5-Phenylbarbitursäure |
| 0,13 | " | Kupfer(II)-chlorid |
| 2,63 | " | Alginat (Manucol FH der Firma Alginat Industries GmbH, Hamburg) |
| 0,2 | " | Natriumpyrophosphat |
| 0,11 | " | Formalin, 37 %ig |

Isolierlösung 1 (Vergleich ohne Puffergemisch)

| | | |
|---|---|---|
| 97,48 | Gewichts-% | Wasser |
| 2,4 | " | Alginat (Manucol FH der Firma Alginat Industries GmbH, Hamburg) |
| 0,12 | " | Formalin, 37 %ig |

<u>Isolierlösung 2</u> (Vergleich ohne Konservierungsmittel)

| | | |
|---|---|---|
| 95,5 | Gewichts-% | Wasser |
| 0,6 | " | Natriumhydroxid |
| 1,0 | " | Citronensäure |
| 0,1 | " | Kupfer(II)-chlorid |
| 2,6 | " | Alginat (Manucol FH der Firma Alginat Industries GmbH, Hamburg) |
| 0,2 | " | Natriumpyrophosphat |

Es werden die Änderungen des pH-Wertes und der Viskosität (bestimmt als Ausflußzeit in Sekunden, gemessen mit dem Ford-Becher) der in den Beispielen 2, 3, 4, 7, 8 und 9 beschriebenen Isoliermittel und der Isolierlösungen 1 (ohne Puffergemisch) und 2 (ohne Konservierungsmittel) in Abhängigkeit von der Zeit untersucht. Die pH-Wert- und Viskositätsänderungen in Abhängigkeit von der Zeit werden in den Figuren 1 bis 8 graphisch dargestellt. Der Versuch mit der kein Konservierungsmittel enthaltenden Isolierlösung 2 mußte nach 6 Wochen wegen Schimmelbildung abgebrochen werden.

0166856

Hanau, 26. Juni 1984
ZPL-Pr/ha

Kulzer & Co. GmbH

Patentanmeldung

"Isoliermittel"

## Patentansprüche

1. Isoliermittel aus einer ein Konservierungsmittel enthaltenden wässerigen Alginat-Lösung, dadurch gekennzeichnet, daß es ein Puffergemisch enthält und einen pH-Wert zwischen 5,5 und 8,5 besitzt.

2. Isoliermittel nach Anspruch 1, dadurch gekennzeichnet, daß das Puffergemisch aus Citronensäure und Natriumhydroxid besteht.

3. Isoliermittel nach Anspruch 1, dadurch gekennzeichnet, daß das Puffergemisch aus Glycin und Natriumhydroxid besteht.

4. Isoliermittel nach Anspruch 1, dadurch gekennzeichnet, daß das Puffergemisch aus Dinatriumhydrogenphosphat-dihydrat und Kaliumdihydrogenphosphat besteht.

5. Isoliermittel nach einem der Ansprüche 1 bis 4, dadurch gekennzeichnet, daß es zusätzlich ein wasserlösliches Kupfer(II)-Salz enthält.

6. Isoliermittel nach einem der Ansprüche 1 bis 4, dadurch gekennzeichnet, daß es zusätzlich ein wasserlösliches Kupfer(II)-Salz und ein wasserlösliches Salz der Barbitursäure oder eines Barbitursäure-Derivats enthält.

7. Isoliermittel nach Anspruch 6, dadurch gekennzeichnet, daß es das Natriumsalz der 5-Phenylbarbitursäure enthält.

8. Isoliermittel nach einem der Ansprüche 5 bis 7, dadurch gekennzeichnet, daß es Kupfer(II)-chlorid enthält.

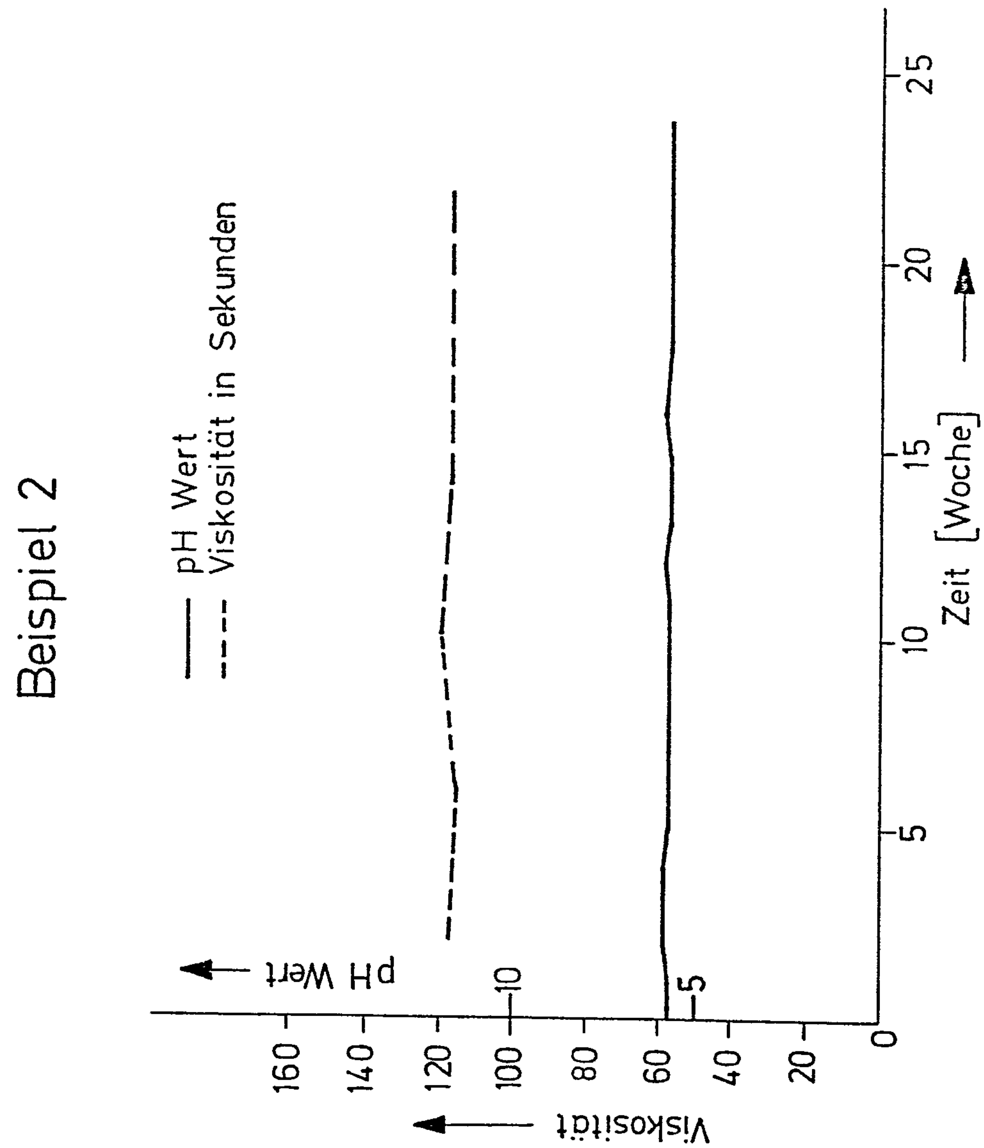
Beispiel 2
pH Wert
Viskosität in Sekunden
Viskosität
pH Wert
160
140
120
100
80
60
40
20
0
10
5
5
10
15
20
25
Zeit [Woche]

Fig. 1

Beispiel 3

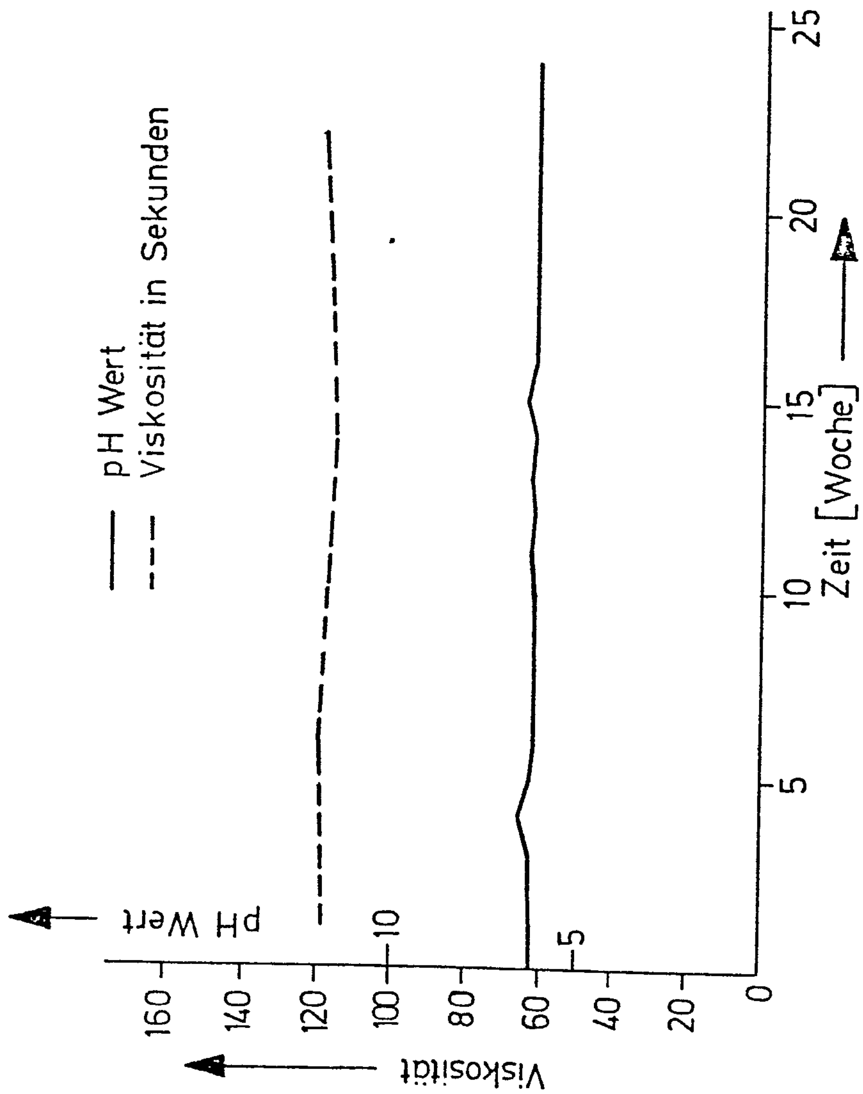


Fig. 2

Beispiel 4

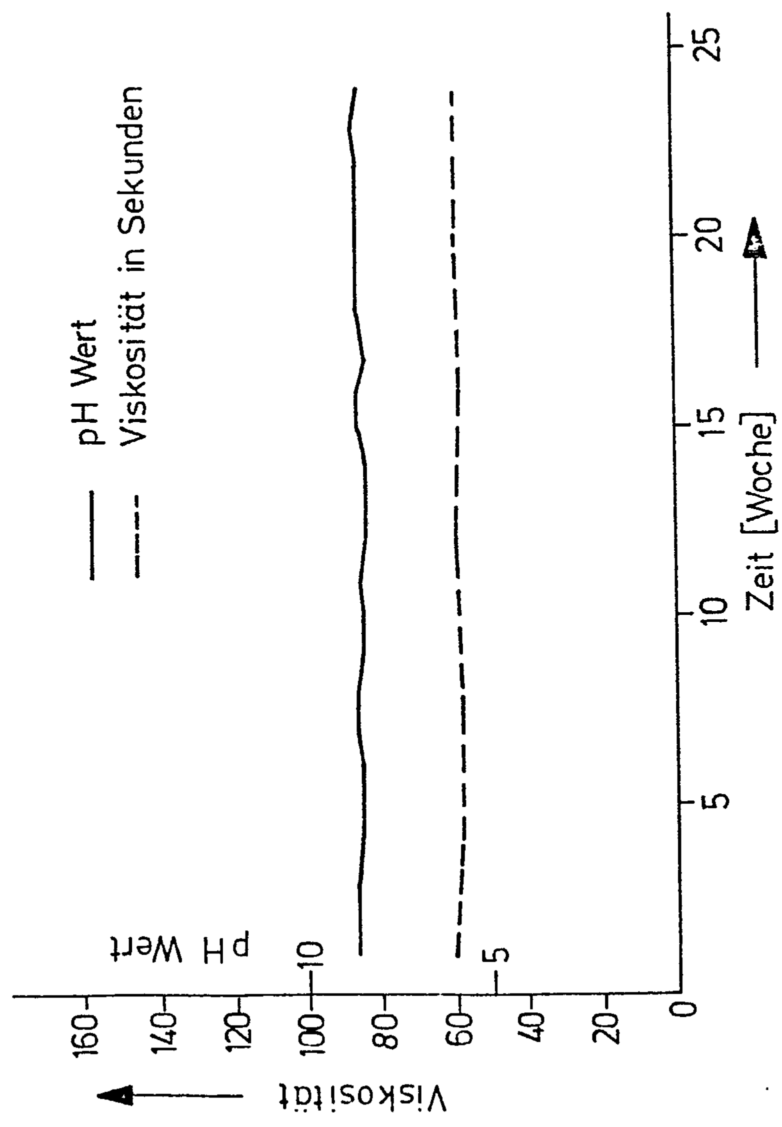


Fig. 3

Beispiel 7

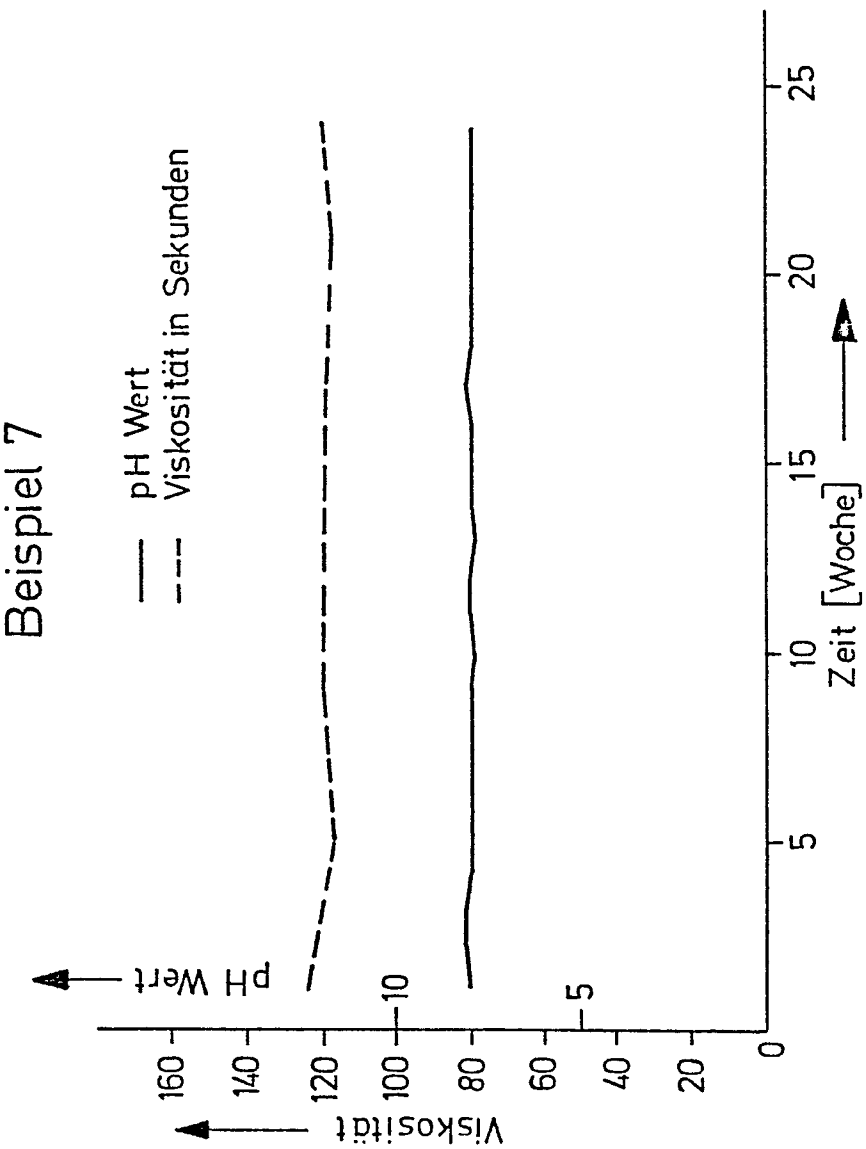


Fig. 4

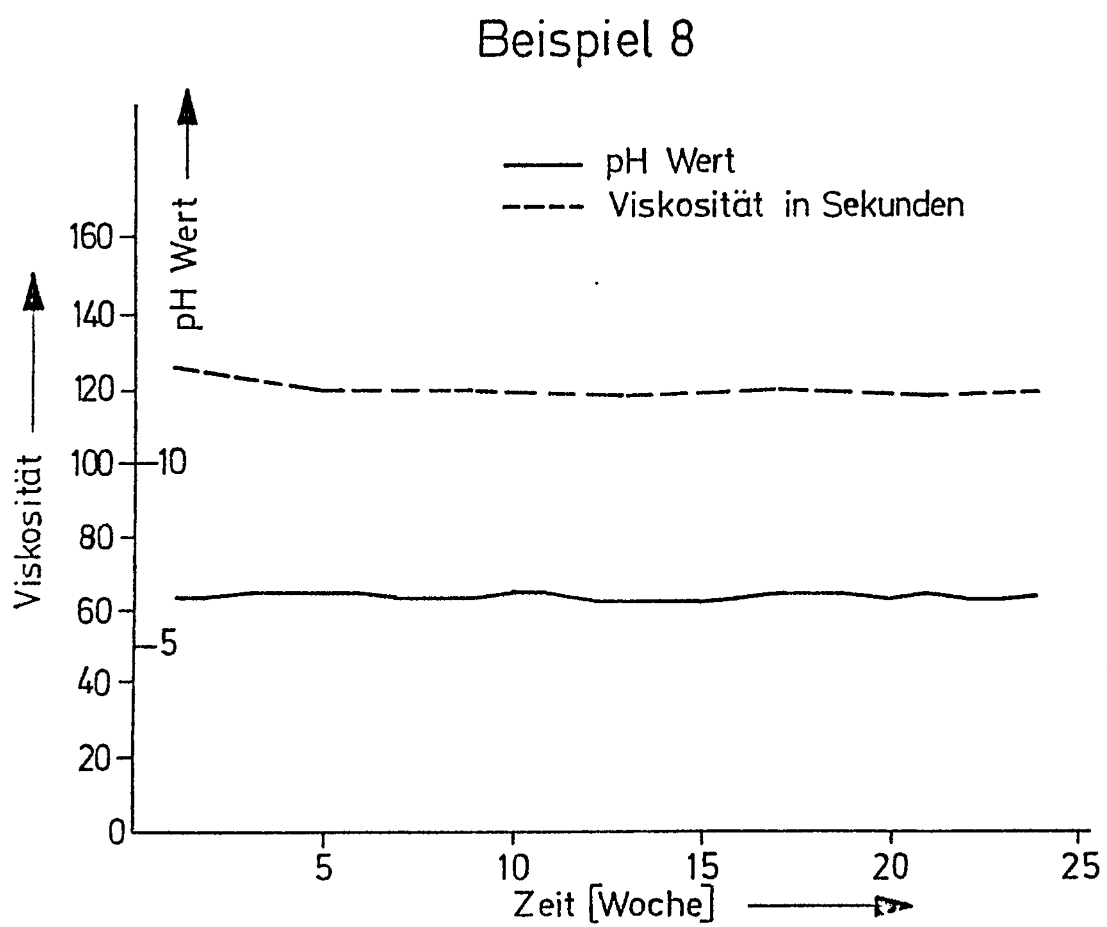
Beispiel 8
pH Wert
Viskosität in Sekunden
pH Wert
Viskosität
160
140
120
100
80
60
40
20
0
10
5
5
10
15
20
25
Zeit [Woche]

Fig. 5

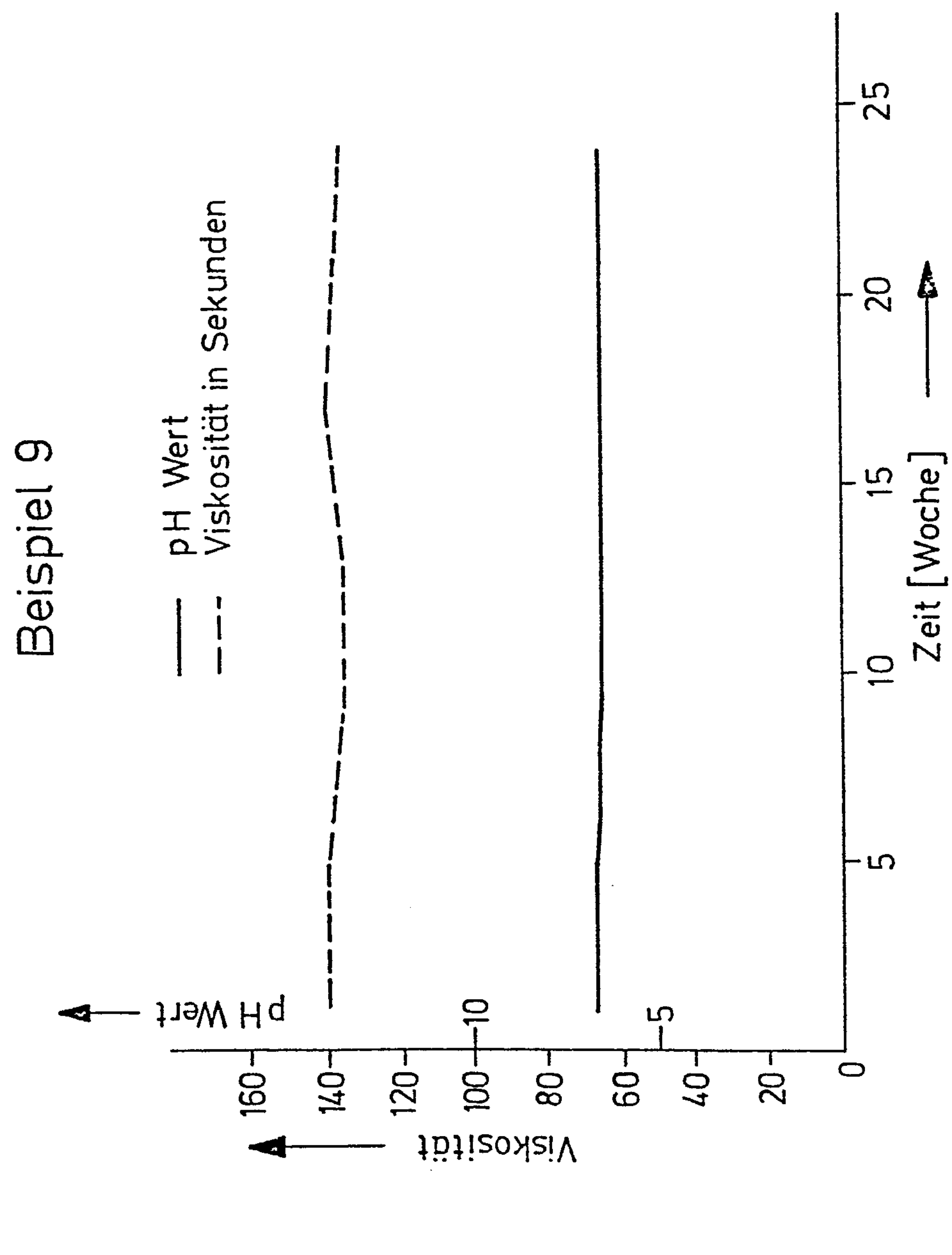
Beispiel 9
pH Wert
Viskosität in Sekunden
pH Wert
Viskosität
10
5
160
140
120
100
80
60
40
20
0
5
10
15
20
25
Zeit [Woche]

Fig. 6

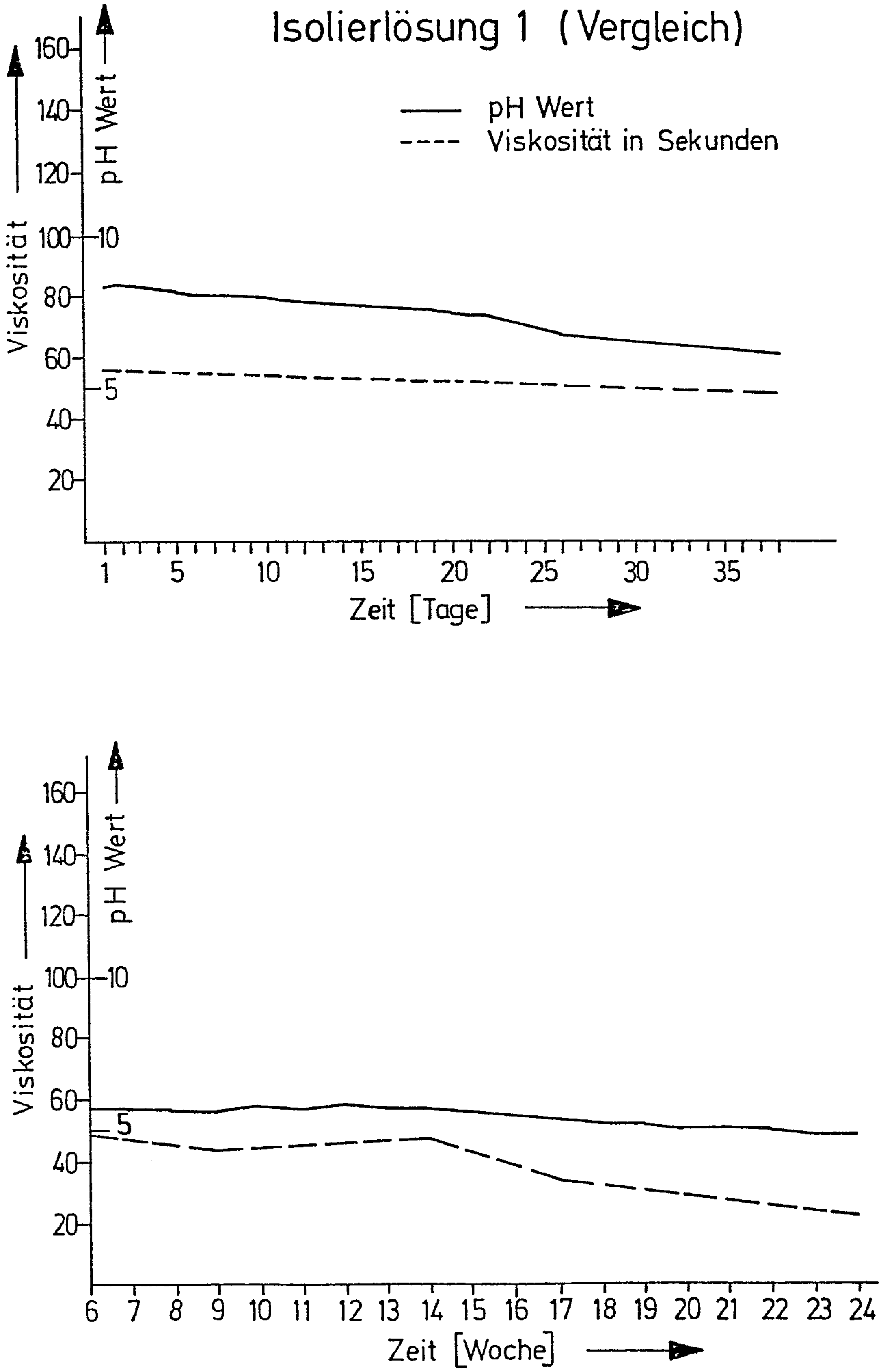
Isolierlösung 1 (Vergleich)
pH Wert
Viskosität in Sekunden
Viskosität
pH Wert
160
140
120
100
80
60
40
20
10
5
1 5 10 15 20 25 30 35
Zeit [Tage]
Viskosität
pH Wert
6 7 8 9 10 11 12 13 14 15 16 17 18 19 20 21 22 23 24
Zeit [Woche]

Fig. 8

Isolierlösung 2 (Vergleich)

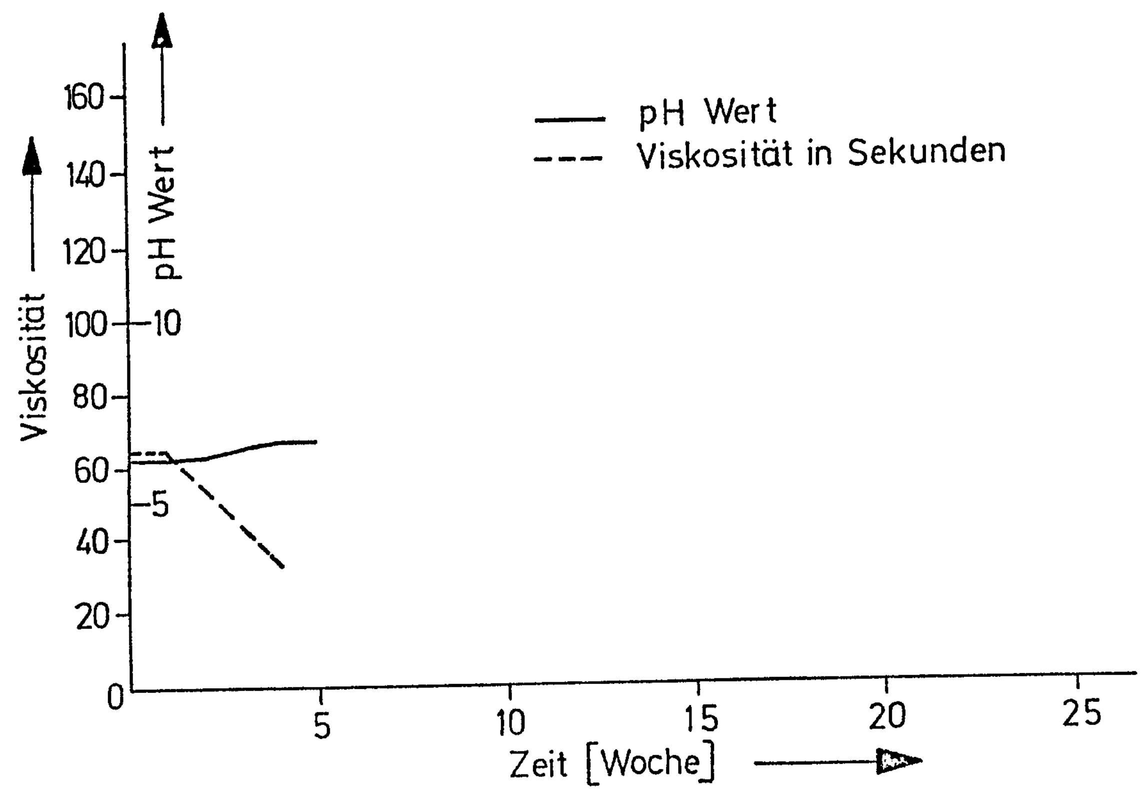


Fig. 7

9/9

0166856

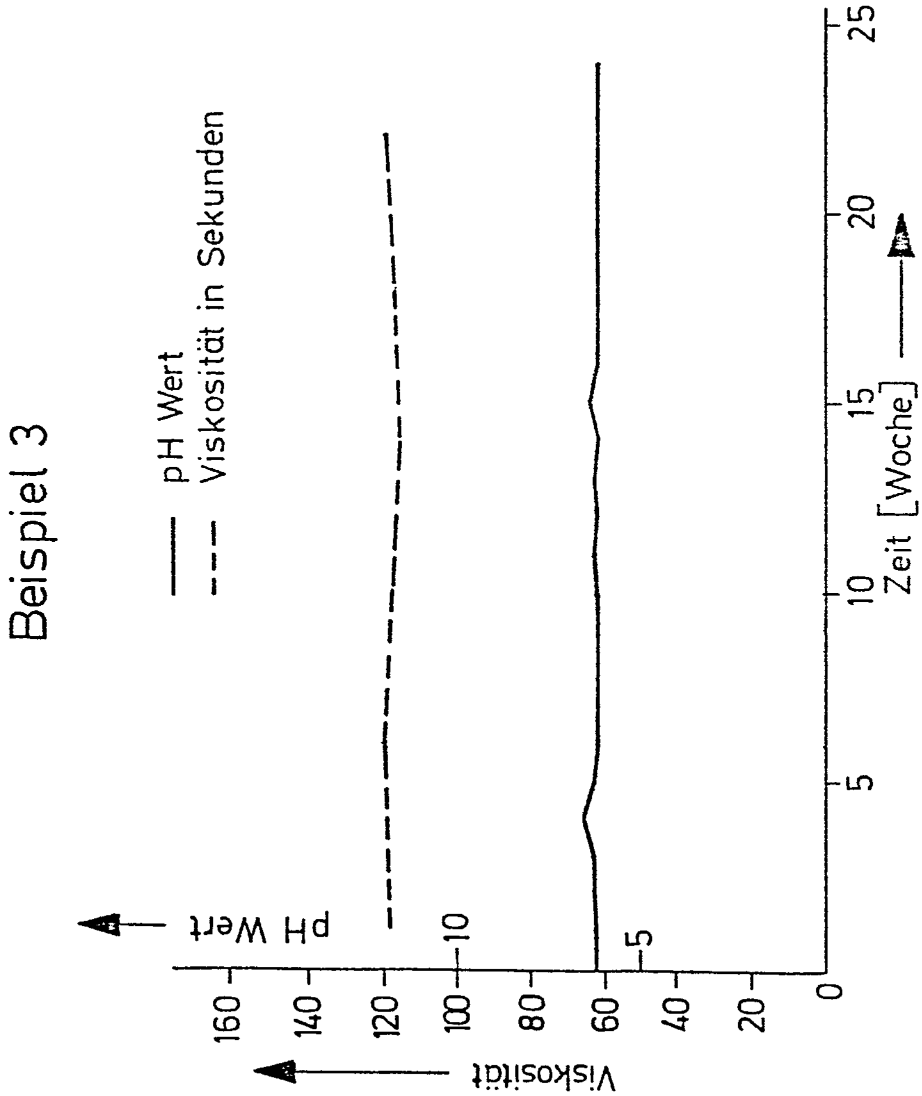


Fig. 2

Europäisches Patentamt

**EUROPÄISCHER RECHERCHENBERICHT**

**0166856**
Nummer der Anmeldung

EP 85 10 2074

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,X<br>Y | CHEMICAL ABSTRACTS, Band 68, 1968, Seite 610, Nr. 6163f, Columbus, Ohio, US; M. BALABANOV u.a.: "Algisol as an insulating alginate solution" & STOMATOLOGIYA (SOFIA) 49(4), 344-53<br>* Zusammenfassung *<br>--- | 1-5,8 | A 61 K 6/02<br>A 61 K 6/00 |
| D,X<br>Y | CHEMICAL ABSTRACTS, Band 96, Nr. 14, April 1982, Seite 392, Nr. 110192d, Columbus, Ohio, US; & RO - A - 71 677 (INTREPRINDEREA "NAPOCHIM") 08.08.1980<br>* Zusammenfassung *<br>--- | 1 | |
| X,Y | CHEMICAL ABSTRACTS, Band 99, Nr. 26, Dezember 1983, Seite 384, Nr. 218591j, Columbus, Ohio, US; & JP - A - 58 145 792 (SADAHIKO IEDA) 30.08.1983<br>* Zusammenfassung *<br>--- | 1,3,4 | RECHERCHIERTE SACHGEBIETE (Int. Cl.4)<br>A 61 K 6/00 |
| Y | US-A-2 432 688 (F.A. SACK)<br>* Ansprüche *<br>--- | 1,2 | |
| Y | US-A-3 681 489 (T.R. FORD)<br>* Ansprüche; Spalte 4, Zeilen 52-65 *<br>----- | 1,5,8 | |

Der vorliegende Recherchenbericht wurde für alle Patentansprüche erstellt.

| Recherchenort | Abschlußdatum der Recherche | Prüfer |
|---|---|---|
| DEN HAAG | 25-06-1985 | SCHURMANS H.D.R. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument